# EUROPEAN PATENT APPLICATION

(11) **EP 4 570 820 A1**
(43) Date of publication of application: **18.06.2025**
(21) Application number: 23851872.4
(22) Date of filing: 09.08.2023
(51) Int. Cl.: C07K 14/62, A61K 38/28, A61P 3/10

(54) **LONG-ACTING INSULIN COMPOUND**

(30) Priority: 10.08.2022 CN 202210956232
(71) Applicant: Chengdu Aoda Biotechnology Co., Ltd., Chengdu, Sichuan 610041 (CN)
(72) Inventor: ZHOU, Shuliang, Chengdu, Sichuan 610041 (CN); WANG, Peng, Chengdu, Sichuan 610041 (CN); DENG, Lan, Chengdu, Sichuan 610041 (CN)
(74) Representative: PGA S.p.A., Milano, Succursale di Lugano
(86) International application number: PCT/CN2023/111935
(87) International publication number: WO 2024/032651

(57) **Abstract**

The present invention relates to the field of pharmaceutical synthesis. A long-acting insulin compound is disclosed. The long-acting insulin compound of the present invention for preparing a pharmaceutical composition for treating disease, and use of the pharmaceutical composition in treating various diseases, including type-I diabetes, type-II diabetes, and gestational diabetes.

## Description

### CROSS REFERENCE TO RELATED APPLICATIONS

This application claims the priority of Chinese Patent Application No. 202210956232.9, filed with the China National Intellectual Property Administration on August 10, 2022, and titled with "LONG-ACTING INSULIN COMPOUND", and the disclosure of which is hereby incorporated by reference in its entirety.

### FIELD

The present invention relates to the field of pharmaceutical synthesis, in particular to a long-acting insulin compound.

### BACKGROUND

Insulin is a protein hormone secreted by pancreatic β cells in the pancreas under the stimulation of endogenous or exogenous substances such as glucose, lactose, ribose, arginine and glucagon. Insulin is the only hormone in the body that lowers blood sugar and promotes the synthesis of glycogen, fat, and protein. Exogenous insulin is mainly used to treat diabetes.

Unlike other recombinant protein drugs, insulin, as a hormone that regulates blood sugar, has a very narrow therapeutic window, and its blood concentration must be strictly controlled. If it is too high, hypoglycemia or even shock and death may occur, and if it is too low, hyperglycemia may occur. Therefore, insulin cannot be injected intravenously and can only be slowly released into the blood through subcutaneous injection. In addition, the sugar intake of three meals a day requires insulin regulation during meals, and the basal blood sugar is regulated by basal insulin. Therefore, the physiological insulin curve is extremely complicated, which is the purposes of designing rapid-acting insulin and long-acting insulin.

Insulin drugs are generally designed into 100IU/ml preparations, and at this concentration, insulin usually forms dimers and then hexamers. After subcutaneous injection of insulin, the hexamer will gradually release dimers and monomers, which enter the blood to take effect. Since insulin takes 4-6 hours to work, which is longer than the mealtime insulin, hypoglycemia is easy to occur 2 hours after a meal. In addition, it acts for too short compared to basal insulin. Protamine can bind with insulin and form a precipitate, which will cause insulin to be slowly dissolved and released after subcutaneous injection, and the action time will be extended to more than ten hours, so that it is generally injected twice a day. Although protamine-insulin has partially solved the problem, it is still far from the insulin secretion curve in the physiological state, and it is determined to be a suspension due to its precipitation properties, for which it is difficult to ensure accurate dose control.

Insulin glargine from Sanofi has a similar sustained release effect as that of the protamine. The addition of two basic amino acids - arginine at the end of the B chain causes the isoelectric point of insulin glargine to change from 5.4 to 6.7. After subcutaneous injection, the insulin glargine is close to the isoelectric point and forms a precipitate, and it is slowly dissolved and released into the blood. However, the insulin glargine must be made into an acidic preparation to be soluble due to the change in its isoelectric point. The asparagine at A21, which is prone to deamidation, is mutated into glycine, which is also the origin of the name of insulin glargine. The half-life of insulin glargine is 12 hours, the duration of its action is 20-24 hours, and it is injected once a day.

Novo Nordisk has developed a new generation of long-acting insulin degludec, which was successfully launched in 2013. In insulin degludec, the threonine at position B30 is removed and a 16-carbon fatty acid chain is connected to the lysine at position B29 through a glutamic acid linker. The fatty acid chain can bind to albumin in plasma and extend the half-life of insulin. This mechanism is the same as that of insulin detemir. In addition, by optimizing the ratio of linker, fatty acid chain and zinc ions, insulin degludec is in the form of a double hexamer in the preparation. After subcutaneous injection, phenol diffuses and insulin degludec undergoes conformational changes, rapidly assembling from the double hexamers into a linear multi-hexamer, generally more than several thousand molecules long. The multi-hexamer slowly releases hexamer, dimer, and monomer which will enter the blood to take effect. Through these two mechanisms, the half-life of insulin degludec reaches 24 hours and the duration of action is up to 42 hours.

Although the above-mentioned long-acting insulin achieves the purpose of once-daily administration, it cannot achieve a longer acting time, for example, once-weekly administration.

### SUMMARY

In view of this, the present invention provides a long-acting insulin compound.

In order to achieve the above-mentioned purpose the present invention provides the following technical solution.

The present invention provides a compound, comprising:
(I) an amino acid $equence set forth in Formula I, 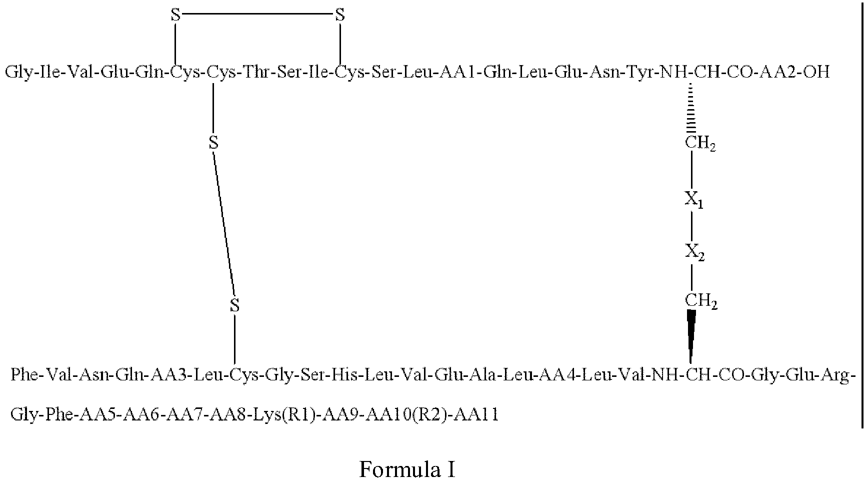 wherein,
   X₁ in Formula I is selected from the group consisting of S, CH₂, NH and CO;
   X₂ in Formula I is selected from the group consisting of S, CH₂, NH and CO;
   AA1 in Formula I is selected from the group consisting of Asp, Glu and Ada;
   AA2 in Formula I is selected from the group consisting of any amino acid for coding other than Cys, and any non-coded amino acid without SH group, or is absent;
   AA3 in Formula I is selected from the group consisting of His, Tyr and Phe;
   AA4 in Formula I is selected from the group consisting of Asp, Glu and Ada;
   AA5 in Formula I is selected from the group consisting of His, Tyr and Phe;
   AA6 in Formula I is selected from the group consisting of any amino acid for coding other than Cys, and any non-coded amino acid without SH group;
   AA7 in Formula I is selected from the group consisting of any amino acid for coding other than Cys, and any non-coded amino acid without SH group;
   AA8 in Formula I is selected from the group consisting of any amino acid for coding other than Cys, and any non-encodable amino acid without SH group;
   AA9 in Formula I is Thr, or is absent;
   AA10 in Formula I is selected from the group consisting of Lys, Dah, Orn, Dab and Dap, or is absent;
   R1 and R2 in Formula I are HO₂C(CH₂)n1CO-(γGlu)n2-(PEGn3(CH₂)n4CO)n5-; and
   R1 and R2 in Formula I are not present at the same time;
(II) an amino acid sequence obtained from substitution, deletion, addition and/or replacement of one or more amino acids of the amino acid sequence set forth in (I) ; or
(III) a sequence with more than 90% homology to the amino acid sequence set forth in (I) or (II); or
(IV) a pharmaceutically acceptable salt, solvate, chelate or non-covalent complex of the compound set forth in Formula I; and/or
(V) a drug precursor based on the compound shown in Formula I; and/or
(VI) a mixture comprising (I), (II), (III), (IV) and/or (V).

In some specific embodiments of the present invention,
when X₁ in Formula I is S, X₂ is S or CH₂;
when X₁ in Formula I is CH₂, X₂ is S or CH₂;
when X₁ in Formula I is NH, X₂ is CO;
when X₁ in Formula I is CO, X₂ is NH;
when X₁ and X₂ in Formula I are both S, AA11 is NH₂; and
when X₁ and X₂ in Formula I are not both S or both are not S, AA11 is NH₂ or OH.

In some specific embodiments of the present invention,
n1 is an integer selected from 10 to 25;
n2 is an integer selected from 1 to 5;
n3 is an integer selected from 1 to 30;
n4 is an integer selected from 1 to 5; and
n5 is an integer selected from 1 to 5.

In some specific embodiments of the present invention, the pharmaceutically acceptable salt includes a complex formed by the compound and Zn²⁺.

The present invention further provides a method for producing the compound, comprising:
Step 1: performing solid-phase polypeptide synthesis to obtain a peptide; and
Step 2: performing acid hydrolysis and purification to obtain the compound.

The present invention further provides use of any one of the following in the manufacture of a medicament or drug combination for preventing and/or treating a disease:
(I) the compound; and/or
(II) the compound produced by the method.

The present invention further provides use of any one of the following in the manufacture of a medicament or drug combination for preventing and/or treating diabetes:
(I) the compound; and/or
(II) the compound produced by the method;
   wherein the diabetes includes type I diabetes, type II diabetes and/or gestational diabetes.

The present invention further provides a drug, comprising the compound and/or the compound produced by the method, and an acceptable excipient and/or adjuvants.

The present invention further provides a drug combination, comprising any one of the following and an additional active ingredient;
(I) the compound; and/or
(II) the compound produced by the method; and/or
(III) the drug.

The present invention further provides a method for preventing and/or treating diabetes, comprising administering any one of the following to a subject in need thereof:
(I) the compound; and/or
(II) the compound produced by the method; and/or
(III) the drug; and/or
(IV) the drug combination;
   wherein the diabetes includes type I diabetes, type II diabetes and/or gestational diabetes.

The present invention includes but is not limited to the following beneficial effects:
providing a long-acting insulin compound with a longer half-life, which has broad application prospects in the treatment of type I diabetes, type II diabetes and gestational diabetes.

### DETAILED DESCRIPTION

The present invention discloses a long-acting insulin compound, and those skilled in the art can refer to the content herein and appropriately improve the process parameters to achieve it. It should be particularly noted that all similar substitutions and modifications are obvious to those skilled in the art, which are all considered to be included in the present invention. The method and application of the present invention have been described through preferred embodiments, and relevant personnel can obviously modify or appropriately change and combine the methods and applications described herein without departing from the content, spirit and scope of the present invention to achieve and apply the technology of the present invention.

The purpose of the present invention is to provide a long-acting insulin compound with a longer half-life.

The present invention provides a long-acting insulin compound and use thereof.

To achieve the above purpose, the present invention first provides a compound represented by Formula I, a pharmaceutically acceptable salt, solvate, chelate, non-covalent complex, or drug precursor thereof, or any mixture of the above forms. 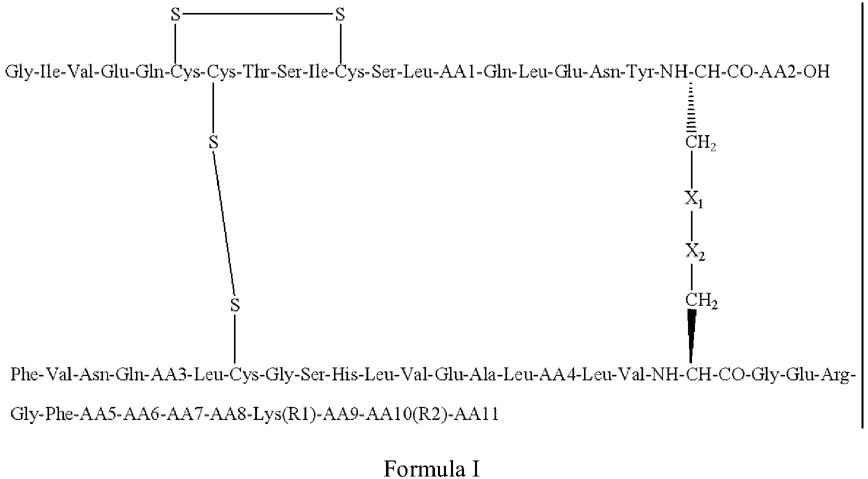
wherein, when X₁ in Formula I is S, X₂ is S or CH₂;
when X₁ in Formula I is CH₂, X₂ is S or CH₂;
when X₁ in Formula I is NH, X₂ is CO;
when X₁ in Formula I is CO, X₂ is NH;
AA1 in Formula I is selected from the group consisting of Asp, Glu and Ada;
AA2 in Formula I is selected from the group consisting of any amino acid for coding other than Cys, and any non-coded amino acid without SH group, or is absent;
AA3 in Formula I is selected from the group consisting of His, Tyr and Phe;
AA4 in Formula I is selected from the group consisting of Asp, Glu and Ada;
AA5 in Formula I is selected from the group consisting of His, Tyr and Phe;
AA6 in Formula I is selected from the group consisting of any amino acid for coding other than Cys, and any non-coded amino acid without SH group;
AA7 in Formula I is selected from the group consisting of any amino acid for coding other than Cys, and any non-coded amino acid without SH group;
AA8 in Formula I is selected from the group consisting of any amino acid for coding other than Cys, and any non-encodable amino acid without SH group;
AA9 in Formula I is Thr, or is absent;
AA10 in Formula I is selected from the group consisting of Lys, Dah, Orn, Dab and Dap, or is absent;
when X₁ and X₂ in Formula I are both S, AA11 is NH₂;
when X₁ and X₂ in Formula I are not both S or both are not S, AA11 is NH₂ or OH
R1 and R2 in Formula I are HO₂C(CH₂)n1CO-(γGlu)n2-(PEGn3(CH₂)n4CO)n5-;
wherein: n1 is an integer selected from 10 to 25;
n2 is an integer selected from 1 to 5;
n3 is an integer selected from 1 to 30;
n4 is an integer selected from 1 to 5;
n5 is an integer selected from 1 to 5; and
R1 and R2 in Formula I are not present at the same time.

The present invention also provides a complex formed by the compound according to the present invention and Zn²⁺.

The present invention further provides a pharmaceutical composition comprising the complex formed by the compound with Zn²⁺, and use of the pharmaceutical composition of the present invention in the manufacture of a pharmaceutical composition for treating a disease.

The present invention further provides use of the pharmaceutical composition in treating various diseases, including type I diabetes, type II diabetes and gestational diabetes.

More contents of the present invention are described in detail below, or some can also be understood in the embodiments of the present invention. Unless otherwise specified, the quantities of different components and reaction conditions used herein can be interpreted as "approximately" or "about" in any case. Accordingly, unless otherwise specified, the numerical parameters cited below and in the claims are approximate parameters, and different numerical parameters may be obtained under respective experimental conditions due to different standard errors.

In the present application, when there is a disagreement or ambiguity between the chemical formula and the chemical name of a compound, the chemical formula is used to accurately define the compound. The compounds described herein may contain one or more chiral centers, and/or double bonds and the like, and may also exist as stereoisomers, including double bond isomers (such as geometric isomers), optical enantiomers or diastereomers. Accordingly, any chemical structure within the scope of the description herein, whether it contains the above-mentioned similar structure in part or in its entirety, includes all possible enantiomers and diastereomers of the compound, including any single stereoisomer (such as a single geometric isomer, a single enantiomer or a single diastereomer) and any mixture of these isomers. These mixtures of racemic isomers and stereoisomers can also be further separated into their constituent enantiomers or stereoisomers by those skilled in the art using continuous resolution techniques or chiral molecular synthesis methods.

The compounds of formula I include, but are not limited to, optical isomers, racemates and/or other mixtures of these compounds. In the above case, a single enantiomer or diastereomer, such as an optically active isomer, can be obtained by asymmetric synthesis or racemate resolution. The resolution of the racemate can be achieved by various methods, such as conventional recrystallization with a reagent that assists the resolution, or by chromatography. In addition, the compound of Formula I also includes cis and/or trans isomers with double bonds.

The compounds described in the present invention include, but are not limited to, the compound represented by Formula I and all of the various pharmaceutically acceptable forms thereof, including various pharmaceutically acceptable salts, solvates, complexes, chelates, non-covalent complexes, prodrugs based on the above substances, and any mixture of the above forms.

The present invention further provides a production method comprising: producing a peptide resin using solid phase peptide synthesis method, subjecting the peptide resin to acid hydrolysis to obtain a crude product, and finally purifying the crude product to obtain a pure product; wherein the step of producing a peptide resin using solid phase peptide synthesis method is performed by sequentially connecting the corresponding protected amino acids or fragments in the sequence to the carrier resin using solid phase coupling synthesis method to obtain the peptide resin.

In the above production method, the amount of the Fmoc-protected amino acid or protected amino acid fragment is 1.2 to 6 times the total molar number of the resin in the reaction, preferably 2.5 to 3.5 times.

In the above production method, the resin substitution is 0.2 to 1.0 mmol/g resin, preferably, the resin substitution is 0.3 to 0.5 mmol/g resin.

As a preferred embodiment of the present invention, the solid phase coupling synthesis method is performed by removing the Fmoc protecting group from the protected amino acid-resin obtained in the previous step, and then coupling the resulting product with the next protected amino acid; wherein, the removing Fmoc protecting group is performed for 10 to 60 minutes, preferably 15 to 25 minutes; and the coupling is performed for 60 to 300 minutes, preferably 100 to 140 minutes.

A condensation reagent is added in the coupling reaction, which is selected from the group consisting of DIC (N,N-diisopropylcarbodiimide), N,N-dicyclohexylcarbodiimide, benzotriazole-1-yl-oxytripyrrolidinophosphine hexafluorophosphate, 2-(7-aza-1H-benzotriazole-1-yl)-1,1,3,3-tetramethyluronium hexafluorophosphate, benzotriazole-N,N,N',N'-tetramethyluronium hexafluorophosphate and O-benzotriazole-N,N,N',N'-tetramethyluronium tetrafluoroborate; preferably N,N-diisopropylcarbodiimide, wherein, the molar number of the condensation reagent is 1.2 to 6 times the total molar number of amino groups in the amino resin, preferably 2.5 to 3.5 times.

An activation reagent is added in the coupling reaction, which is selected from the group consisting of 1-hydroxybenzotriazole and N-hydroxy-7-azabenzotriazole, preferably 1-hydroxybenzotriazole; wherein, the amount of the activation reagent is 1.2 to 6 times the total molar number of amino groups in the amino resin, preferably 2.5 to 3.5 times.

As a preferred embodiment of the present invention, the Fmoc-removing reagent is a mixed solution of PIP/DMF (piperidine/N,N-dimethylformamide), and the piperidine content in the mixed solution is 10 to 30% (V); the amount of the Fmoc-removing reagent is 5 to 15 mL per gram of amino resin, preferably 8 to 12 mL per gram of amino resin.

Preferably, the peptide resin is subjected to acid hydrolysis to remove the resin and side chain protecting groups to obtain a crude product.

Further preferably, the acid hydrolysis agent used in the acid hydrolysis of peptide resin is a mixed solvent of trifluoroacetic acid (TFA), 1,2-ethanedithiol (EDT) and water; and the volume ratio of the mixed solvent is 80 to 95% of TFA, 1 to 10% of EDT and water for the balance.

Further preferably, the volume ratio of the mixed solvent is 89 to 91% of TFA, 4 to 6% of EDT and water for the balance. Optimally, the volume ratio of the mixed solvent is 90% of TFA, 5% of EDT and water for the balance.

The amount of the acid hydrolysis agent used is 4 to 15 mL of acid hydrolysis agent per gram of peptide resin; preferably, the amount of acid hydrolysis agent used is 7 to 10 mL per gram of peptide resin.

The acid hydrolysis is performed for 1 to 6 hours at room temperature, preferably 3 to 4 hours.

Further, the crude product is purified by high performance liquid chromatography and freeze-dried to obtain a pure product.

The terms for the abbreviations involved in the present invention are shown in the Table 1:

**Table 1 Terms and abbreviations**

| Abbreviation | Term | Abbreviation | Term |
|---|---|---|---|
| Fmoc | 9-Fluorenylmethoxy carbonyl | OtBu | tert-Butoxy |
| tBu | tert-Butyl | Boc | tert-Butoxycarbonyl |
| Trt | Trityl | Pbf | (2,3-dihydro-2,2,4,6,7-pentamethylben zofuran-5-yl)sulfonyl |
| Ala | Alanine | Leu | Leucine |
| Arg | Arginine | Lys | Lysine |
| Asn | Asparagine | Met | Methionine |
| Asp | Aspartic acid | Phe | Phenylalanine |
| Cys | Cysteine | Pro | Proline |
| Gln | Glutamine | Ser | Serine |
| Glu | Glutamic acid | Thr | Threonine |
| Gly | Glycine | Trp | Tryptophan |
| His | Histidine | Tyr | Tyrosine |
| Ile | Isoleucine | Val | Valine |
| Dap | 2,3-diaminopropioni c acid | Dab | 2,4-diaminobutyric acid |
| Orn | Ornithine | Dah | 2,7-diaminoheptanoic acid |
| Acm | S-acetyl | DAS | (S,S)-2,7-diaminooctanedioic acid |
| Mtt | 4-methyltrityl | Ada | 2-aminoadipic acid |

The raw materials and reagents used in the production of long-acting insulin compound provided by the present invention were all commercially available.

The present invention is further described below in conjunction with examples.

### Example 1 Production of Compound 1

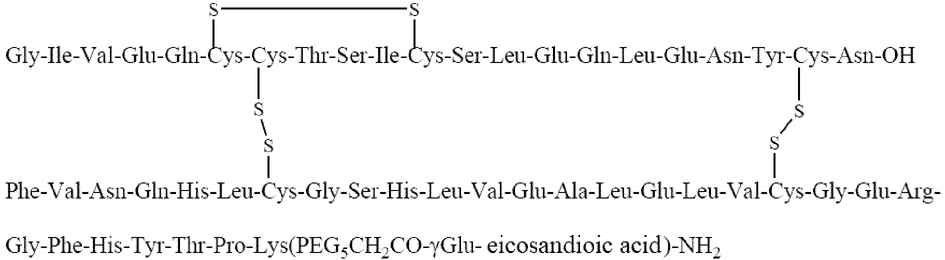

wherein,
Gly-Ile-Val-Glu-Gln-Cys-Cys-Thr-Ser-Ile-Cys-Ser-Leu-Glu-Gln-Leu-Glu-Asn-Tyr-Cys -Asn-OH is set forth in SEQ ID NO: 1;
Phe-Val-Asn-Gln-His-Leu-Cys-Gly-Ser-His-Leu-Val-Glu-Ala-Leu-Glu-Leu-Val-Cys-Gl y-Glu-Arg-Gly-Phe-His-Tyr-Thr-Pro-Lys(PEG₃CH₂CO-γGlu-eicosandioic acid)-NH₂ is set forth in SEQ ID NO: 2.

### 1. Synthesis of peptide resin

Rink Amide BHHA resin was used as the carrier resin, and coupled with the protected amino acids shown in the following table in sequence through removing Fmoc protection and coupling reaction to obtain a peptide resin. The protected amino acids used in this example and the corresponding protected amino acids are shown in Table 2:

**Table 2 Protected amino acids used in Example 1**

| Order in the peptide n= | Protected amino acids |
|---|---|
| 1 | Fmoc-Lys(PEG₅CH₂CO-γGlu(OtBu)- 20-(tert-butoxy)-20-oxoicosanoic acid) |
| 2 | Fmoc-Pro |
| 3 | Fmoc-Thr(tBu) |
| 4 | Fmoc-Tyr(tBu) |
| 5 | Fmoc-His(Trt) |
| 6 | Fmoc-Phe |
| 7 | Fmoc-Gly |
| 8 | Fmoc-Arg(Pbf) |
| 9 | Fmoc-Glu(OtBu) |
| 10 | Fmoc-Gly |
| 11 | Fmoc-Cys(Mtt) |
| 12 | Fmoc-Val |
| 13 | Fmoc-Leu |
| 14 | Fmoc-Glu(OtBu) |
| 15 | Fmoc-Leu |
| 16 | Fmoc-Ala |
| 17 | Fmoc-Glu(OtBu) |
| 18 | Fmoc-Val |
| 19 | Fmoc-Leu |
| 20 | Fmoc-His(Trt) |
| 21 | Fmoc-Ser(tBu) |
| 22 | Fmoc-Gly |
| 23 | Fmoc-Cys(Trt) |
| 24 | Fmoc-Leu |
| 25 | Fmoc-His(Trt) |
| 26 | Fmoc-Gln(Trt) |
| 27 | Fmoc-Asn(Trt) |
| 28 | Fmoc-Val |
| 29 | Boc-Phe |
| Side chain 1 | Fmoc-Cys-Asn(Trt)-OtBu |
| Side chain 2 | Fmoc-Tyr(tBu) |
| Side chain 3 | Fmoc-Asn(Trt) |
| Side chain 4 | Fmoc-Glu(OtBu) |
| Side chain 5 | Fmoc-Leu |
| Side chain 6 | Fmoc-Gln(Trt) |
| Side chain 7 | Fmoc-Glu(OtBu) |
| Side chain 8 | Fmoc-Leu |
| Side chain 9 | Fmoc-Ser(tBu) |
| Side chain 10 | Fmoc-Cys(Acm) |
| Side chain 11 | Fmoc-Ile |
| Side chain 12 | Fmoc-Ser(tBu) |
| Side chain 13 | Fmoc-Thr(tBu) |
| Side chain 14 | Fmoc-Cys(Trt) |
| Side chain 15 | Fmoc-Cys(Acm) |
| Side chain 16 | Fmoc-Gln(Trt) |
| Side chain 17 | Fmoc-Glu(OtBu) |
| Side chain 18 | Fmoc-Val |
| Side chain 19 | Fmoc-Ile |
| Side chain 20 | Boc-Gly |

### (1) Coupling with the first protected amino acid in the main chain

0.03 mmol of the first protected amino acid and 0.03 mmol of HOBt were dissolved in an appropriate amount of DMF; 0.03 mmol of DIC was slowly added into the solution of protected amino acid in DMF under stirring, and the reaction was carried out at room temperature for 30 min with stirring to obtain an activated protected amino acid solution for standby use.

0.01 mmol of Rink amide MBHA resin (with a resin substitution of about 0.3 mmol/g) was deprotected using 20% PIP/DMF solution for 25 minutes, washed and filtered to obtain a resin without Fmoc protection.

The solution of the activated first protected amino acid was added to the resin without Fmoc, and subjected to coupling reaction for 60 to 300 minutes, filtered and washed to obtain a resin coupling with one protected amino acid.

### (2) Coupling with the 2nd to 29th protected amino acids in the main chain

The corresponding 2nd to 29th protected amino acids were sequentially connected, using the same method as for coupling the first protected amino acid in the main chain to obtain a resin containing 29 amino acids in the main chain.

### (3) Coupling with the first protected amino acid in the side chain

The protection of the side chain was removed using 50% HFIP/DCM solution, repeated 5 times, 35 minutes each time, filtered and washed to obtain a resin without Mtt protection for later use.

0.3 mol of 2,2'-dithiodipyridine was dissolved in an appropriate amount of DMF, and the mixture was added to the above-mentioned resin without Mtt protection, stirred and reacted for 3 hours, filtered and washed to obtain a SH-activated resin for later use.

0.03 mol of the first protected amino acid in the side chain (Fmoc-Cys-Asn(Trt)-OtBu), was dissolved in an appropriate amount of DMF, and the mixture was added to the above-mentioned SH-activated resin, added with 2 ml of DIPEA, stirred and reacted for 3 hours, filtered and washed to complete the connection of the first amino acid in the side chain.

### (4) Coupling with the second protected amino acid in the side chain

0.03 mol of the second protected amino acid in the side chain and 0.03 mol of HOBt were dissolved in an appropriate amount of DMF; 0.03 mol of DIC was slowly added to the solution of the protected amino acid in DMF under stirring, and the mixture was stirred and reacted at room temperature for 30 minutes to obtain an activated solution of protected amino acid.

Deprotection was performed using 20% PIP/DMF solution for 25 minutes, and the mixture was washed and filtered. The activated second protected amino acid solution in the side chain was added to the resin without Fmoc protection, and the coupling reaction was performed for 60 to 300 minutes. The resulting mixture was filtered and washed to obtain a resin containing the second protected amino acid in the side chain.

### (5) Coupling with the 3rd to 20th protected amino acids in the side chain

The corresponding 3rd to 20th protected amino acids were connected to the side chain in sequence using the same method as the coupling of the first protected amino acid in the main chain to obtain a peptide resin.

### 2. Production of crude product

The above peptide resin was added with a cleavage reagent with a volume ratio of TFA:Tis:water = 95:5:5 at 10mL of cleavage reagent/g resin, stirred evenly, and reacted at room temperature for 3 hours. The reaction mixture was filtered with a sand core funnel, the filtrate was collected, and the resin was washed with a small amount of TFA 3 times. The filtrates were combined and concentrated under reduced pressure, and added with anhydrous ether for precipitation. The obtained precipitate was washed with anhydrous ether 3 times, and dried by suction to obtain an off-white powder.

The obtained off-white powder was dissolved in 20% DMSO aqueous solution, adjusted to pH 7.5 with ammonia water, stirred for 10 hours of reaction, then added with glacial acetic acid to reach 20% acetic acid, added with iodine/ethanol saturated solution dropwise under stirring until complete cyclization, and concentrated under reduced pressure at 35-40°C to obtain a concentrated solution of crude product.

### 3. Production of pure product

The above crude concentrated solution was filtered with a mixed membrane filter with a pore size of 0.45µm, and purified for later use.

The purification was performed using high performance liquid chromatography, wherein the packing material of the chromatographic column for purification was reverse phase C18 with a column particle size of 10µm, the mobile phase system was 0.1% TFA/water solution-0.1% TFA/acetonitrile solution, the flow rate of the 30mm×250mm chromatographic column was 20mL/min, a gradient system elution was adopted, and cyclic loading was used for purification. The solution of the crude product was loaded on the chromatographic column, the mobile phase elution was started, and the main peak was collected and evaporated to remove acetonitrile to obtain a purified intermediate concentrate.

The purified intermediate concentrate was filtered with a 0.45 µm filter membrane for standby use. The salt was exchanged by high performance liquid chromatography. The mobile phase system was 1% acetic acid/water solution-acetonitrile, and the packing material of the chromatographic column for purification was reverse phase C18 with a column particle size of 10µm. The flow rate of the 30 mm × 250 mm chromatographic column was 20 mL/min (the flow rate could be adjusted according to the different specifications of the chromatographic column); a gradient elution and cyclic loading method were used, the sample was loaded into the chromatographic column, the mobile phase elution was started, the spectrum was collected, and the change of absorbance was observed. The main peak fraction of salt exchange was collected, and the purity thereof was detected by analytical liquid phase. The main peak fractions of salt exchange were combined, and concentrated under reduced pressure to obtain an aqueous solution of pure product in acetic acid, which was freeze-dried to obtain 3.9g of pure product with a purity of 97.7% and a total yield of 6.1%. The molecular weight of the pure product is 6358.2 (100% M+H).

### Example 2 Production of Compound 2

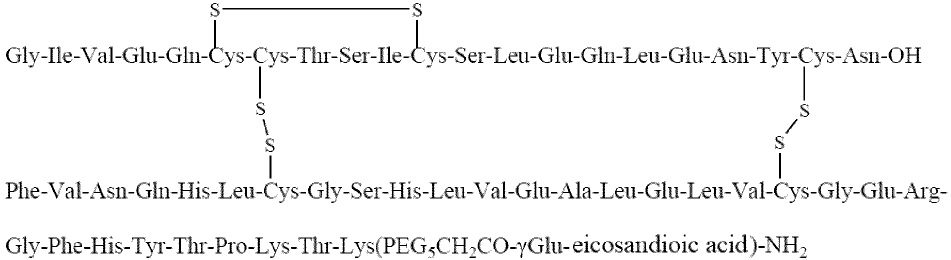

wherein,
Gly-Ile-Val-Glu-Gln-Cys-Cys-Thr-Ser-Ile-Cys-Ser-Leu-Glu-Gln-Leu-Glu-Asn-Tyr-Cys -Asn-OH is set forth in SEQ ID NO: 1;
Phe-Val-Asn-Gln-His-Leu-Cys-Gly-Ser-His-Leu-Val-Glu-Ala-Leu-Glu-Leu-Val-Cys-Gl y-Glu-Arg-Gly-Phe-His-Tyr-Thr-Pro-Lys-Thr-Lys(PEG₃CH₂CO-γGlu-eicosandioic acid)-NH₂ is set forth in SEQ ID NO: 3.

The production method is the same as that of Example 1, and the protected amino acids used are shown in Table 3 below:

**Table 3 Protected amino acids used in Example 2**

| Order in the peptide n= | Protected amino acids |
|---|---|
| 1 | Fmoc-Lys(PEG₅CH₂CO-γGlu(OtBu)- 20-(tert-butoxy)-20-oxoicosanoic acid) |
| 2 | Fmoc-Thr(tBu) |
| 3 | Fmoc-Lys(Boc) |
| 4 | Fmoc-Pro |
| 5 | Fmoc-Thr(tBu) |
| 6 | Fmoc-Tyr(tBu) |
| 7 | Fmoc-His(Trt) |
| 8 | Fmoc-Phe |
| 9 | Fmoc-Gly |
| 10 | Fmoc-Arg(Pbf) |
| 11 | Fmoc-Glu(OtBu) |
| 12 | Fmoc-Gly |
| 13 | Fmoc-Cys(Mtt) |
| 14 | Fmoc-Val |
| 15 | Fmoc-Leu |
| 16 | Fmoc-Glu(OtBu) |
| 17 | Fmoc-Leu |
| 18 | Fmoc-Ala |
| 19 | Fmoc-Glu(OtBu) |
| 20 | Fmoc-Val |
| 21 | Fmoc-Leu |
| 22 | Fmoc-His(Trt) |
| 23 | Fmoc-Ser(tBu) |
| 24 | Fmoc-Gly |
| 25 | Fmoc-Cys(Trt) |
| 26 | Fmoc-Leu |
| 27 | Fmoc-His(Trt) |
| 28 | Fmoc-Gln(Trt) |
| 29 | Fmoc-Asn(Trt) |
| 30 | Fmoc-Val |
| 31 | Boc-Phe |
| Side chain 1 | Fmoc-Cys-Asn(Trt)-OtBu |
| Side chain 2 | Fmoc-Tyr(tBu) |
| Side chain 3 | Fmoc-Asn(Trt) |
| Side chain 4 | Fmoc-Glu(OtBu) |
| Side chain 5 | Fmoc-Leu |
| Side chain 6 | Fmoc-Gln(Trt) |
| Side chain 7 | Fmoc-Glu(OtBu) |
| Side chain 8 | Fmoc-Leu |
| Side chain 9 | Fmoc-Ser(tBu) |
| Side chain 10 | Fmoc-Cys(Acm) |
| Side chain 11 | Fmoc-Ile |
| Side chain 12 | Fmoc-Ser(tBu) |
| Side chain 13 | Fmoc-Thr(tBu) |
| Side chain 14 | Fmoc-Cys(Trt) |
| Side chain 15 | Fmoc-Cys(Acm) |
| Side chain 16 | Fmoc-Gln(Trt) |
| Side chain 17 | Fmoc-Glu(OtBu) |
| Side chain 18 | Fmoc-Val |
| Side chain 19 | Fmoc-Ile |
| Side chain 20 | Boc-Gly |

4.7g of pure product was obtained, with a purity of 98.5% and a total yield of 7.1%. The molecular weight of the pure product is 6587.5 (100% M+H).

### Example 3 Production of Compound 3

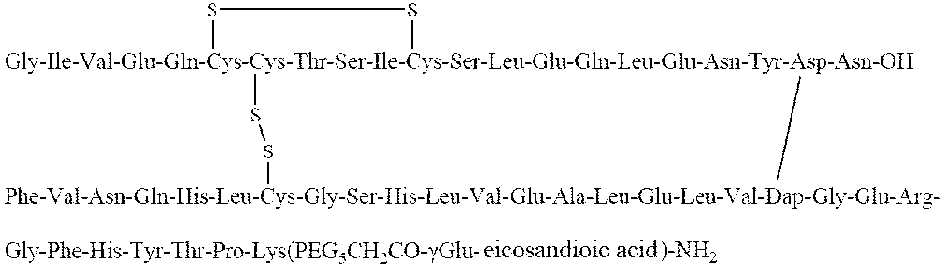

wherein,
Gly-Ile-Val-Glu-Gln-Cys-Cys-Thr-Ser-Ile-Cys-Ser-Leu-Glu-Gln-Leu-Glu-Asn-Tyr-Asp -Asn-OH is set forth in SEQ ID NO: 4;
Phe-Val-Asn-Gln-His-Leu-Cys-Gly-Ser-His-Leu-Val-Glu-Ala-Leu-Glu-Leu-Val-Dap-G ly-Glu-Arg-Gly-Phe-His-Tyr-Thr-Pro-Lys(PEG₃CH₂CO-γGlu-eicosandioic acid)-NH₂ is set forth in SEQ ID NO: 5.

(1) The production method was the same as Example 1 except for the coupling of the first protected amino acid in the side chain.
(2) Coupling with the first protected amino acid in the side chain

0.03 mol of the first protected amino acid in the side chain and 0.03 mol of HOBt were dissolved in an appropriate amount of DMF; 0.03 mol of DIC was slowly added to the solution of the protected amino acid in DMF under stirring, and the resulting mixture was stirred and reacted at room temperature for 30 minutes to obtain an activated protected amino acid solution.

The protection of the side chain was removed using 50% HFIP/DCM solution, repeated 5 times, 35 minutes each time, washed and filtered, and the activated solution of the first protected amino acid in the side chain was added to the resin without Mtt protection, and subjected to coupling reaction for 60 to 300 minutes, filtered and washed to obtain a resin containing the first protected amino acid in the side chain.

The protected amino acids used are shown in Table 4 below:

**Table 4 Protected amino acids used in Example 3**

| Order in the peptide n= | Protected amino acids |
|---|---|
| 1 | Fmoc-Lys(PEG₅CH₂CO-γGlu(OtBu)-20-(tert-butoxy)-20-oxoicosanoic acid) |
| 2 | Fmoc-Pro |
| 3 | Fmoc-Thr(tBu) |
| 4 | Fmoc-Tyr(tBu) |
| 5 | Fmoc-His(Trt) |
| 6 | Fmoc-Phe |
| 7 | Fmoc-Gly |
| 8 | Fmoc-Arg(Pbf) |
| 9 | Fmoc-Glu(OtBu) |
| 10 | Fmoc-Gly |
| 11 | Fmoc-Dap(Mtt) |
| 12 | Fmoc-Val |
| 13 | Fmoc-Leu |
| 14 | Fmoc-Glu(OtBu) |
| 15 | Fmoc-Leu |
| 16 | Fmoc-Ala |
| 17 | Fmoc-Glu(OtBu) |
| 18 | Fmoc-Val |
| 19 | Fmoc-Leu |
| 20 | Fmoc-His(Trt) |
| 21 | Fmoc-Ser(tBu) |
| 22 | Fmoc-Gly |
| 23 | Fmoc-Cys(Trt) |
| 24 | Fmoc-Leu |
| 25 | Fmoc-His(Trt) |
| 26 | Fmoc-Gln(Trt) |
| 27 | Fmoc-Asn(Trt) |
| 28 | Fmoc-Val |
| 29 | Boc-Phe |
| Side chain 1 | Fmoc-Asp-Asn(Trt)-OtBu |
| Side chain 2 | Fmoc-Tyr(tBu) |
| Side chain 3 | Fmoc-Asn(Trt) |
| Side chain 4 | Fmoc-Glu(OtBu) |
| Side chain 5 | Fmoc-Leu |
| Side chain 6 | Fmoc-Gln(Trt) |
| Side chain 7 | Fmoc-Glu(OtBu) |
| Side chain 8 | Fmoc-Leu |
| Side chain 9 | Fmoc-Ser(tBu) |
| Side chain 10 | Fmoc-Cys(Acm) |
| Side chain 11 | Fmoc-Ile |
| Side chain 12 | Fmoc-Ser(tBu) |
| Side chain 13 | Fmoc-Thr(tBu) |
| Side chain 14 | Fmoc-Cys(Trt) |
| Side chain 15 | Fmoc-Cys(Acm) |
| Side chain 16 | Fmoc-Gln(Trt) |
| Side chain 17 | Fmoc-Glu(OtBu) |
| Side chain 18 | Fmoc-Val |
| Side chain 19 | Fmoc-Ile |
| Side chain 20 | Boc-Gly |

3.5g of pure product was obtained, with a purity of 98.8% and a total yield of 5.5%. The molecular weight of the pure product is 6337.2 (100% M+H).

### Example 4 Production of Compound 4

The structure of Compound 4 is as follows (SEQ ID NO: 4): 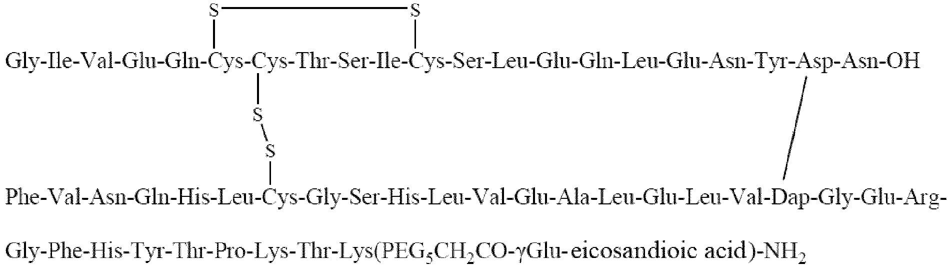 wherein,
Gly-Ile-Val-Glu-Gln-Cys-Cys-Thr-Ser-Ile-Cys-Ser-Leu-Glu-Gln-Leu-Glu-Asn-Tyr-Asp -Asn-OH is set forth in SEQ ID NO: 4;
Phe-Val-Asn-Gln-His-Leu-Cys-Gly-Ser-His-Leu-Val-Glu-Ala-Leu-Glu-Leu-Val-Dap-G ly-Glu-Arg-Gly-Phe-His-Tyr-Thr-Pro-Lys-Thr-Lys(PEG₅CH₂CO-γGlu-eicosandioic acid)-NH₂ is set forth in SEQ ID NO: 6.

The production method was the same as in Example 3, and the protected amino acids used are shown in Table 5 below:

**Table 5 Protected amino acids used in Example 4**

| Order in the peptide n= | Protected amino acids |
|---|---|
| 1 | Fmoc-Lys(PEG₅CH₂CO-γGlu(OtBu)-20-(tert-butoxy)-20-oxoicosanoic acid) |
| 2 | Fmoc-Thr(tBu) |
| 3 | Fmoc-Lys(Boc) |
| 4 | Fmoc-Pro |
| 5 | Fmoc-Thr(tBu) |
| 6 | Fmoc-Tyr(tBu) |
| 7 | Fmoc-His(Trt) |
| 8 | Fmoc-Phe |
| 9 | Fmoc-Gly |
| 10 | Fmoc-Arg(Pbf) |
| 11 | Fmoc-Glu(OtBu) |
| 12 | Fmoc-Gly |
| 13 | Fmoc-Dap(Mtt) |
| 14 | Fmoc-Val |
| 15 | Fmoc-Leu |
| 16 | Fmoc-Glu(OtBu) |
| 17 | Fmoc-Leu |
| 18 | Fmoc-Ala |
| 19 | Fmoc-Glu(OtBu) |
| 20 | Fmoc-Val |
| 21 | Fmoc-Leu |
| 22 | Fmoc-His(Trt) |
| 23 | Fmoc-Ser(tBu) |
| 24 | Fmoc-Gly |
| 25 | Fmoc-Cys(Trt) |
| 26 | Fmoc-Leu |
| 27 | Fmoc-His(Trt) |
| 28 | Fmoc-Gln(Trt) |
| 29 | Fmoc-Asn(Trt) |
| 30 | Fmoc-Val |
| 31 | Boc-Phe |
| Side chain 1 | Fmoc-Asp-Asn(Trt)-OtBu |
| Side chain 2 | Fmoc-Tyr(tBu) |
| Side chain 3 | Fmoc-Asn(Trt) |
| Side chain 4 | Fmoc-Glu(OtBu) |
| Side chain 5 | Fmoc-Leu |
| Side chain 6 | Fmoc-Gln(Trt) |
| Side chain 7 | Fmoc-Glu(OtBu) |
| Side chain 8 | Fmoc-Leu |
| Side chain 9 | Fmoc-Ser(tBu) |
| Side chain 10 | Fmoc-Cys(Acm) |
| Side chain 11 | Fmoc-Ile |
| Side chain 12 | Fmoc-Ser(tBu) |
| Side chain 13 | Fmoc-Thr(tBu) |
| Side chain 14 | Fmoc-Cys(Trt) |
| Side chain 15 | Fmoc-Cys(Acm) |
| Side chain 16 | Fmoc-Gln(Trt) |
| Side chain 17 | Fmoc-Glu(OtBu) |
| Side chain 18 | Fmoc-Val |
| Side chain 19 | Fmoc-Ile |
| Side chain 20 | Boc-Gly |

3.7g of pure product was obtained, with a purity of 98.1% and a total yield of 5.6%. The molecular weight of the pure product is 6566.4 (100% M+H).

### Example 5 Production of Compound 5

The structure of Compound 5 is shown as follows (SEQ ID NO: 5): 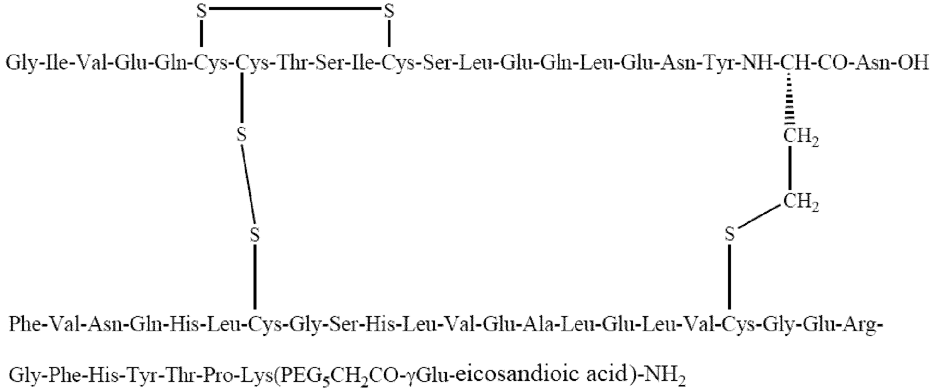 wherein,
Gly-Ile-Val-Glu-Gln-Cys-Cys-Thr-Ser-Ile-Cys-Ser-Leu-Glu-Gln-Leu-Glu-Asn-Tyr-L-4-bromo-2-aminobutyramide-Asn-OH is set forth in SEQ ID NO: 7;
Phe-Val-Asn-Gln-His-Leu-Cys-Gly-Ser-His-Leu-Val-Glu-Ala-Leu-Glu-Leu-Val-Cys-Gl y-Glu-Arg-Gly-Phe-His-Tyr-Thr-Pro-Lys(PEG₅CH₂CO-γGlu-eicosandioic acid)-NH₂ is set forth in SEQ ID NO: 8.

(1) The production method was the same as Example 1 except for the coupling of the first protected amino acid in the side chain.
(2) Coupling with the first protected amino acid in the side chain

The protection of the side chain was removed using 50% HFIP/DCM solution, repeated 5 times, 35 minutes each time, washed and filtered to obtain a resin without Mtt protection for later use.

0.03 mol of the first protected amino acid in the side chain was dissolved with an appropriate amount of DMF, and the resulting mixture was added to the resin without Mtt protection, stirred evenly, then added with 0.03 mol N-methylmorpholine and 0.03 mol lithium chloride, stirred for 6 hours of reaction, filtered and washed to obtain a resin coupling with the first protected amino acid in the side chain.

The protected amino acids used are shown in Table 6 below:

**Table 6 Protected amino acids used in Example 5**

| Order in the peptide n= | Protected amino acids |
|---|---|
| 1 | Fmoc-Lys(PEG₅CH₂CO-γGlu(OtBu)-20-(tert-butoxy)-20-oxoicosan oic acid) |
| 2 | Fmoc-Pro |
| 3 | Fmoc-Thr(tBu) |
| 4 | Fmoc-Tyr(tBu) |
| 5 | Fmoc-His(Trt) |
| 6 | Fmoc-Phe |
| 7 | Fmoc-Gly |
| 8 | Fmoc-Arg(Pbf) |
| 9 | Fmoc-Glu(OtBu) |
| 10 | Fmoc-Gly |
| 11 | Fmoc-Cys(Mtt) |
| 12 | Fmoc-Val |
| 13 | Fmoc-Leu |
| 14 | Fmoc-Glu(OtBu) |
| 15 | Fmoc-Leu |
| 16 | Fmoc-Ala |
| 17 | Fmoc-Glu(OtBu) |
| 18 | Fmoc-Val |
| 19 | Fmoc-Leu |
| 20 | Fmoc-His(Trt) |
| 21 | Fmoc-Ser(tBu) |
| 22 | Fmoc-Gly |
| 23 | Fmoc-Cys(Trt) |
| 24 | Fmoc-Leu |
| 25 | Fmoc-His(Trt) |
| 26 | Fmoc-Gln(Trt) |
| 27 | Fmoc-Asn(Trt) |
| 28 | Fmoc-Val |
| 29 | Boc-Phe |
| Side chain 1 | Fmoc-L-4-bromo-2-aminobutyramide-Asn(Trt)-OtBu |
| Side chain 2 | Fmoc-Tyr(tBu) |
| Side chain 3 | Fmoc-Asn(Trt) |
| Side chain 4 | Fmoc-Glu(OtBu) |
| Side chain 5 | Fmoc-Leu |
| Side chain 6 | Fmoc-Gln(Trt) |
| Side chain 7 | Fmoc-Glu(OtBu) |
| Side chain 8 | Fmoc-Leu |
| Side chain 9 | Fmoc-Ser(tBu) |
| Side chain 10 | Fmoc-Cys(Acm) |
| Side chain 11 | Fmoc-Ile |
| Side chain 12 | Fmoc-Ser(tBu) |
| Side chain 13 | Fmoc-Thr(tBu) |
| Side chain 14 | Fmoc-Cys(Trt) |
| Side chain 15 | Fmoc-Cys(Acm) |
| Side chain 16 | Fmoc-Gln(Trt) |
| Side chain 17 | Fmoc-Glu(OtBu) |
| Side chain 18 | Fmoc-Val |
| Side chain 19 | Fmoc-Ile |
| Side chain 20 | Boc-Gly |

4.3g of pure product was obtained, with a purity of 97.9% and a total yield of 6.8%. The molecular weight of the pure product is 6340.2 (100% M+H).

### Example 6 Production of Compound 6

The structure of Compound 6 is shown as follows (SEQ ID NO: 6): 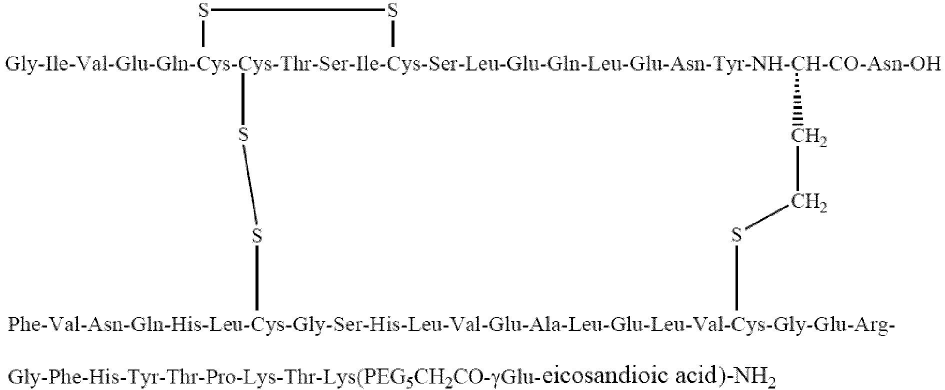 wherein,
Gly-Ile-Val-Glu-Gln-Cys-Cys-Thr-Ser-Ile-Cys-Ser-Leu-Glu-Gln-Leu-Glu-Asn-Tyr-L-4-bromo-2-aminobutyramide-Asn-OH is set forth in SEQ ID NO: 7;
Phe-Val-Asn-Gln-His-Leu-Cys-Gly-Ser-His-Leu-Val-Glu-Ala-Leu-Glu-Leu-Val-Cys-Gl y-Glu-Arg-Gly-Phe-His-Tyr-Thr-Pro-Lys-Thr-Lys(PEG₅CH₂CO-γGlu-eicosandioic acid)-NH₂ is set forth in SEQ ID NO: 9.

The production method was the same as that of Example 5. The protected amino acids used are shown in Table 7 below:

**Table 7 Protected amino acids used in Example 6**

| Order in the peptide n= | Protected amino acids |
|---|---|
| 1 | Fmoc-Lys(PEG₅CH₂CO-γGlu(OtBu)-20-(tert-butoxy)-20-oxoicosanoic acid) |
| 2 | Fmoc-Thr(tBu) |
| 3 | Fmoc-Lys(Boc) |
| 4 | Fmoc-Pro |
| 5 | Fmoc-Thr(tBu) |
| 6 | Fmoc-Tyr(tBu) |
| 7 | Fmoc-His(Trt) |
| 8 | Fmoc-Phe |
| 9 | Fmoc-Gly |
| 10 | Fmoc-Arg(Pbf) |
| 11 | Fmoc-Glu(OtBu) |
| 12 | Fmoc-Gly |
| 13 | Fmoc-Cys(Mtt) |
| 14 | Fmoc-Val |
| 15 | Fmoc-Leu |
| 16 | Fmoc-Glu(OtBu) |
| 17 | Fmoc-Leu |
| 18 | Fmoc-Ala |
| 19 | Fmoc-Glu(OtBu) |
| 20 | Fmoc-Val |
| 21 | Fmoc-Leu |
| 22 | Fmoc-His(Trt) |
| 23 | Fmoc-Ser(tBu) |
| 24 | Fmoc-Gly |
| 25 | Fmoc-Cys(Trt) |
| 26 | Fmoc-Leu |
| 27 | Fmoc-His(Trt) |
| 28 | Fmoc-Gln(Trt) |
| 29 | Fmoc-Asn(Trt) |
| 30 | Fmoc-Val |
| 31 | Boc-Phe |
| Side chain 1 | Fmoc-L-4-bromo-2-aminobutyramide-Asn(Trt)-OtBu |
| Side chain 2 | Fmoc-Tyr(tBu) |
| Side chain 3 | Fmoc-Asn(Trt) |
| Side chain 4 | Fmoc-Glu(OtBu) |
| Side chain 5 | Fmoc-Leu |
| Side chain 6 | Fmoc-Gln(Trt) |
| Side chain 7 | Fmoc-Glu(OtBu) |
| Side chain 8 | Fmoc-Leu |
| Side chain 9 | Fmoc-Ser(tBu) |
| Side chain 10 | Fmoc-Cys(Acm) |
| Side chain 11 | Fmoc-Ile |
| Side chain 12 | Fmoc-Ser(tBu) |
| Side chain 13 | Fmoc-Thr(tBu) |
| Side chain 14 | Fmoc-Cys(Trt) |
| Side chain 15 | Fmoc-Cys(Acm) |
| Side chain 16 | Fmoc-Gln(Trt) |
| Side chain 17 | Fmoc-Glu(OtBu) |
| Side chain 18 | Fmoc-Val |
| Side chain 19 | Fmoc-Ile |
| Side chain 20 | Boc-Gly |

3.5g of pure product was obtained, with a purity of 98.6% and a total yield of 6.4%. The molecular weight of the pure product is 6569.5 (100% M+H).

### Example 7 Production of Compound 7

The structure of Compound 7 is as follows (SEQ ID NO: 7): 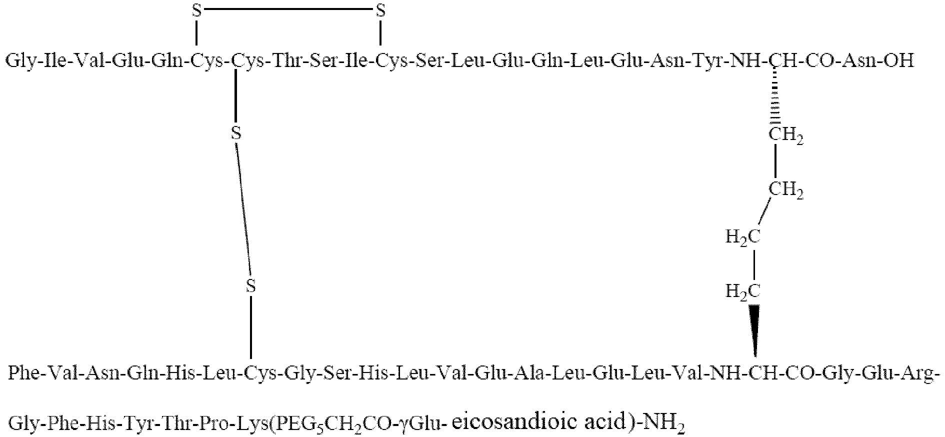 wherein,
Gly-Ile-Val-Glu-Gln-Cys-Cys-Thr-Ser-Ile-Cys-Ser-Leu-Glu-Gln-Leu-Glu-Asn-Tyr-OH is set forth in SEQ ID NO: 10;
Phe-Val-Asn-Gln-His-Leu-Cys-Gly-Ser-His-Leu-Val-Glu-Ala-Leu-Glu-Leu-Val-(S,S) DAS[Dde-Asn(Trt)-OtBu]-Gly-Glu-Arg-Gly-Phe-His-Tyr-Thr-Pro-Lys(PEG₅CH₂CO-γGlu-eicos andioic acid)-NH₂ is set forth in SEQ ID NO: 11.

(1) The production method was the same as Example 1 except for the coupling of the first protected amino acid in the side chain.
(2) Coupling with the first protected amino acid in the side chain

0.03 mol of the first protected amino acid in the side chain and 0.03 mol of HOBt were dissolved in an appropriate amount of DMF; 0.03 mol of DIC was slowly added under stirring to the protected amino acid DMF solution, and the resulting mixture was stirred and reacted at room temperature for 30 minutes to obtain an activated protected amino acid solution.

The protection of the side chain was removed using 2% hydrazine hydrate/DMF solution, repeated 3 times, 10 minutes each time, washed and filtered. The activated solution of the first protected amino acid in the side chain was added to the resin without Dde, subjected to coupling reaction for 60 to 300 minutes, filtered and washed to obtain a resin coupling with the first protected amino acid in the side chain.

The protected amino acids used are shown in Table 8 below:

**Table 8 Protected amino acids used in Example 7**

| Order in the peptide n= | Protected amino acids |
|---|---|
| 1 | Fmoc-Lys(PEG₅CH₂CO-γGlu(OtBu)-20-(tert-butoxy)-20-oxoicosanoic acid) |
| 2 | Fmoc-Pro |
| 3 | Fmoc-Thr(tBu) |
| 4 | Fmoc-Tyr(tBu) |
| 5 | Fmoc-His(Trt) |
| 6 | Fmoc-Phe |
| 7 | Fmoc-Gly |
| 8 | Fmoc-Arg(Pbf) |
| 9 | Fmoc-Glu(OtBu) |
| 10 | Fmoc-Gly |
| 11 | (S,S)-Dde-Asn(Trt)-OtBu-DAS-Fmoc-OH |
| 12 | Fmoc-Val |
| 13 | Fmoc-Leu |
| 14 | Fmoc-Glu(OtBu) |
| 15 | Fmoc-Leu |
| 16 | Fmoc-Ala |
| 17 | Fmoc-Glu(OtBu) |
| 18 | Fmoc-Val |
| 19 | Fmoc-Leu |
| 20 | Fmoc-His(Trt) |
| 21 | Fmoc-Ser(tBu) |
| 22 | Fmoc-Gly |
| 23 | Fmoc-Cys(Trt) |
| 24 | Fmoc-Leu |
| 25 | Fmoc-His(Trt) |
| 26 | Fmoc-Gln(Trt) |
| 27 | Fmoc-Asn(Trt) |
| 28 | Fmoc-Val |
| 29 | Boc-Phe |
| Side chain 1 | Fmoc-Tyr(tBu) |
| Side chain 2 | Fmoc-Asn(Trt) |
| Side chain 3 | Fmoc-Glu(OtBu) |
| Side chain 4 | Fmoc-Leu |
| Side chain 5 | Fmoc-Gln(Trt) |
| Side chain 6 | Fmoc-Glu(OtBu) |
| Side chain 7 | Fmoc-Leu |
| Side chain 8 | Fmoc-Ser(tBu) |
| Side chain 9 | Fmoc-Cys(Acm) |
| Side chain 10 | Fmoc-Ile |
| Side chain 11 | Fmoc-Ser(tBu) |
| Side chain 12 | Fmoc-Thr(tBu) |
| Side chain 13 | Fmoc-Cys(Trt) |
| Side chain 14 | Fmoc-Cys(Acm) |
| Side chain 15 | Fmoc-Gln(Trt) |
| Side chain 16 | Fmoc-Glu(OtBu) |
| Side chain 17 | Fmoc-Val |
| Side chain 18 | Fmoc-Ile |
| Side chain 19 | Boc-Gly |

3.7g of pure product was obtained, with a purity of 98.2% and a total yield of 5.9%. The molecular weight of the pure product is 6322.2 (100% M+H).

### Example 8 Production of Compound 8

The structure of Compound 8 is as follows (SEQ ID NO: 8): 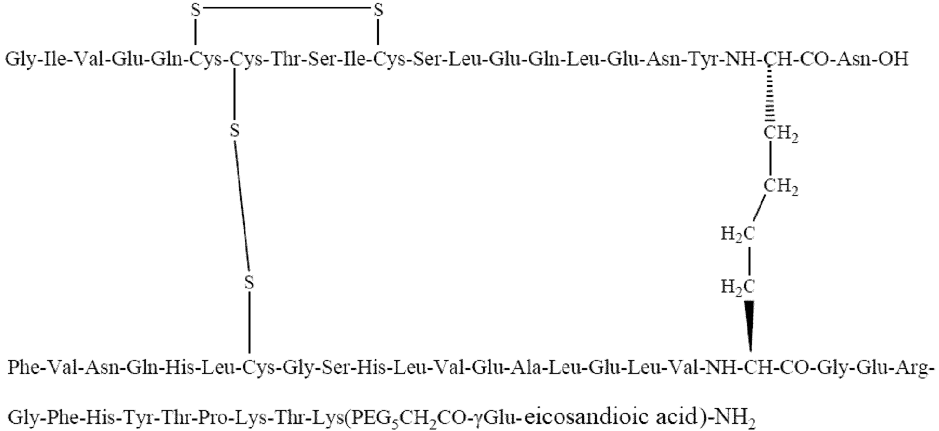 wherein,
Gly-Ile-Val-Glu-Gln-Cys-Cys-Thr-Ser-Ile-Cys-Ser-Leu-Glu-Gln-Leu-Glu-Asn-Tyr-OH is set forth in SEQ ID NO: 10;
Phe-Val-Asn-Gln-His-Leu-Cys-Gly-Ser-His-Leu-Val-Glu-Ala-Leu-Glu-Leu-Val-(S,S) DAS[Dde-Asn(Trt)-OtBu]-Gly-Glu-Arg-Gly-Phe-His-Tyr-Thr-Pro-Lys-Thr-Lys(PEG₃CH₂CO-γ Glu-eicosandioic acid)-NH₂ is set forth in SEQ ID NO: 12.

The production method was the same as in Example 7. The protected amino acids used are shown in Table 9 below:

**Table 9 Protected amino acids used in Example 8**

| Order in the peptide n= | Protected amino acids |
|---|---|
| 1 | Fmoc-Lys(PEG₅CH₂CO-γGlu(OtBu)-20-(tert-butoxy)-20-oxoicosanoic acid) |
| 2 | Fmoc-Thr(tBu) |
| 3 | Fmoc-Lys(Boc) |
| 4 | Fmoc-Pro |
| 5 | Fmoc-Thr(tBu) |
| 6 | Fmoc-Tyr(tBu) |
| 7 | Fmoc-His(Trt) |
| 8 | Fmoc-Phe |
| 9 | Fmoc-Gly |
| 10 | Fmoc-Arg(Pbf) |
| 11 | Fmoc-Glu(OtBu) |
| 12 | Fmoc-Gly |
| 13 | (S,S)-Dde-Asn(Trt)-OtBu-DAS-Fmoc-OH |
| 14 | Fmoc-Val |
| 15 | Fmoc-Leu |
| 16 | Fmoc-Glu(OtBu) |
| 17 | Fmoc-Leu |
| 18 | Fmoc-Ala |
| 19 | Fmoc-Glu(OtBu) |
| 20 | Fmoc-Val |
| 21 | Fmoc-Leu |
| 22 | Fmoc-His(Trt) |
| 23 | Fmoc-Ser(tBu) |
| 24 | Fmoc-Gly |
| 25 | Fmoc-Cys(Trt) |
| 26 | Fmoc-Leu |
| 27 | Fmoc-His(Trt) |
| 28 | Fmoc-Gln(Trt) |
| 29 | Fmoc-Asn(Trt) |
| 30 | Fmoc-Val |
| 31 | Boc-Phe |
| Side chain 1 | Fmoc-Tyr(tBu) |
| Side chain 2 | Fmoc-Asn(Trt) |
| Side chain 3 | Fmoc-Glu(OtBu) |
| Side chain 4 | Fmoc-Leu |
| Side chain 5 | Fmoc-Gln(Trt) |
| Side chain 6 | Fmoc-Glu(OtBu) |
| Side chain 7 | Fmoc-Leu |
| Side chain 8 | Fmoc-Ser(tBu) |
| Side chain 9 | Fmoc-Cys(Acm) |
| Side chain 10 | Fmoc-Ile |
| Side chain 11 | Fmoc-Ser(tBu) |
| Side chain 12 | Fmoc-Thr(tBu) |
| Side chain 13 | Fmoc-Cys(Trt) |
| Side chain 14 | Fmoc-Cys(Acm) |
| Side chain 15 | Fmoc-Gln(Trt) |
| Side chain 16 | Fmoc-Glu(OtBu) |
| Side chain 17 | Fmoc-Val |
| Side chain 18 | Fmoc-Ile |
| Side chain 19 | Boc-Gly |

4.0 g of pure product was obtained with a purity of 97.5% and a total yield of 6.1%. The molecular weight of the pure product is 6551.4 (100% M+H).

### Example 9 Determination of preliminary pharmacokinetic properties

Male SD rats were subcutaneously administered with a dose of 1 mg/kg. Blood samples were collected from the orbital vein of the rats before administration (0 h) and 1h, 2h, 3h, 4h, 8h, 24h, 48h, 96h and 144h after administration, and plasma samples were separated by centrifugation.

The blood drug concentration of the corresponding compound in the plasma samples was determined by liquid chromatography-mass spectrometry. The half-life of the compound in SD rat by subcutaneous (SC) administration is shown in Table 10 below:

**Table 10 Determination of preliminary pharmacokinetic properties**

| **Compound** | **t_{1/2} (h)** |
|---|---|
| Compound 1 | 13.5 |
| Compound 2 | 12.1 |
| Compound 3 | 13.4 |
| Compound 4 | 15.9 |
| Compound 5 | 16.6 |
| Compound 6 | 17.8 |
| Compound 7 | 20.5 |
| Compound 8 | 19.3 |

The above is a detailed description of a long-acting insulin compound provided by the present invention. Specific examples are used herein to illustrate the principles and embodiments of the present invention. The description of the above examples is only used to help understand the method of the present invention and its core idea. It should be noted that for those skilled in the art, without departing from the principle of the present invention, the present invention can also be improved and modified in several ways, and these improvements and modifications also fall within the scope of protection of the claims of the present invention.

SEQUENCE LISTING
<?xml version="1.0" encoding="UTF-8"?><!DOCTYPE ST26SequenceListing SYSTEM
"ST26SequenceListing_V1_3.dtd" PUBLIC "-//WIPO//DTD Sequence Listing
1.3//EN"><ST26SequenceListing productionDate="2023-08-08" softwareVersion="2.1.1"
softwareName="WIPO Sequence" fileName="OP230814.xml" dtdVersion="V1_3"
nonEnglishFreeTextLanguageCode="zh"
originalFreeTextLanguageCode="zh"><ApplicationIdentification><IPOfficeCode>CN</IPOffice
Code><ApplicationNumberText/><FilingDate/></ApplicationIdentification><ApplicantFileRefe
rence>OP230814</ApplicantFileReference><EarliestPriorityApplicationIdentification><IPOffic
eCode>CN</IPOfficeCode><ApplicationNumberText>202210956232.9</ApplicationNumberTe
xt><FilingDate>2022-08-10</FilingDate></EarliestPriorityApplicationIdentification><Applican
tName languageCode="en"> Chengdu Auda Biotechnology Co. ,
Ltd.</ApplicantName><ApplicantNameLatin>Chengdu Auda Biotechnology Co. ,
Ltd.</ApplicantNameLatin><InventionTitle languageCode="en"> LONG-ACTING INSULIN
COMPOUND
</InventionTitle><SequenceTotalQuantity>12</SequenceTotalQuantity><SequenceData
sequenceIDNumber="1"><INSDSeq><INSDSeq_length>21</INSDSeq_length><INSDSeq_mol
type>AA</INSDSeq_moltype><INSDSeq_division>PAT</INSDSeq_division><INSDSeq_featu
re-table><INSDFeature><INSDFeature_key>source</INSDFeature_key><INSDFeature_locatio
n>1..21</INSDFeature_location><INSDFeature_quals><INSDQualifier><INSDQualifier_name
>mol_type</INSDQualifier_name><INSDQualifier_value>protein</INSDQualifier_value></IN
SDQualifier><INSDQualifier
id="q2"><INSDQualifier_name>organism</INSDQualifier_name><INSDQualifier_value>synth
etic
construct</INSDQualifier_value></INSDQualifier></INSDFeature_quals></INSDFeature><IN
SDFeature><INSDFeature_key>DISULFID</INSDFeature_key><INSDFeature_location>6..20
</INSDFeature_location><INSDFeature_quals><INSDQualifier
id="q18"><INSDQualifier_name>note</INSDQualifier_name><INSDQualifier_value>6..11</I
NSDQualifier_value><NonEnglishQualifier_value> The cysteine at position 6 is linked to the
cysteine at position 11 via an intrachain disulfide bond

</NonEnglishQualifier_value></INSDQualifier></INSDFeature_quals></INSDFeature><INSD
Feature><INSDFeature_key>SITE</INSDFeature_key><INSDFeature_location>7..20</INSDFe
ature_location><INSDFeature_quals><INSDQualifier
id="q61"><INSDQualifier_name>note</INSDQualifier_name><INSDQualifier_value>20</INS
DQualifier_value><NonEnglishQualifier_value> The cysteine at position 20 is linked to the
cysteine at position 19 set forth in SEQ ID NO: 2 or SEQ ID NO: 3 through an interchain
disulfide bond </NonEnglishQualifier_value></INSDQualifier><INSDQualifier
id="q60"><INSDQualifier_name>note</INSDQualifier_name><INSDQualifier_value>7</INSD
Qualifier_value><NonEnglishQualifier_value> The cysteine at position 7 is linked to the
cysteine at position 7 set forth in SEQ ID NO: 2 or SEQ ID NO: 3 through an interchain disulfide
bond
</NonEnglishQualifier_value></INSDQualifier></INSDFeature_quals></INSDFeature></INSD
Seq_feature-table><INSDSeq_sequence>GIVEQCCTSICSLEQLENYCN</INSDSeq_sequence
></INSDSeq></SequenceData><SequenceData
sequenceIDNumber="2"><INSDSeq><INSDSeq_length>29</INSDSeq_length><INSDSeq_mol
type>AA</INSDSeq_moltype><INSDSeq_division>PAT</INSDSeq_division><INSDSeq_featu
re-table><INSDFeature><INSDFeature_key>source</INSDFeature_key><INSDFeature_locatio
n>1..29</INSDFeature_location><INSDFeature_quals><INSDQualifier><INSDQualifier_name
>mol_type</INSDQualifier_name><INSDQualifier_value>protein</INSDQualifier_value></IN
SDQualifier><INSDQualifier
id="q3"><INSDQualifier_name>organism</INSDQualifier_name><INSDQualifier_value>synth
etic
construct</INSDQualifier_value></INSDQualifier></INSDFeature_quals></INSDFeature><IN
SDFeature><INSDFeature_key>SITE</INSDFeature_key><INSDFeature_location>7..19</INS
DFeature_location><INSDFeature_quals><INSDQualifier
id="q58"><INSDQualifier_name>note</INSDQualifier_name><INSDQualifier_value>7</INSD
Qualifier_value><NonEnglishQualifier_value> The cysteine at position 7 is linked to the
cystcinc at position 7 sct forth in SEQ ID NO: 1 through an intcrchain disulfidc bond
</NonEnglishQualifier_value></INSDQualifier><INSDQualifier
id="q59"><INSDQualifier_name>note</INSDQualifier_name><INSDQualifier_value>19</INS

DQualifier_value><NonEnglishQualifier_value> The cysteine at position 19 is linked to the
cysteine at position 20 set forth in SEQ ID NO: 1 through an interchain disulfide bond
</NonEnglishQualifier_value></INSDQualifier></INSDFeature_quals></INSDFeature><INSD
Feature><INSDFeature_key>MOD_RES</INSDFeature_key><INSDFeature_location>29</INS
DFeature_location><INSDFeature_quals><INSDQualifier
id="q16"><INSDQualifier_name>note</INSDQualifier_name><INSDQualifier_value>29</INS
DQualifier_value><NonEnglishQualifier_value> Lysine modified with PEG5CH2CO-gamma
Glu-eicosanedioic acid
</NonEnglishQualifier_value></INSDQualifier></INSDFeature_quals></INSDFeature></INSD
Seq_feature-table><INSDSeq_sequence>FVNQHLCGSHLVEALELVCGERGFHYTPX</INSD
Seq_sequence></INSDSeq></SequenceData><SequenceData
sequenceIDNumber="3"><INSDSeq><INSDSeq_length>31</INSDSeq_length><INSDSeq_mol
type>AA</INSDSeq_moltype><INSDSeq_division>PAT</INSDSeq_division><INSDSeq_featu
re-table><INSDFeature><INSDFeature_key>source</INSDFeature_key><INSDFeature_locatio
n>1..31</INSDFeature_location><INSDFeature_quals><INSDQualifier><INSDQualifier_name
>mol_type</INSDQualifier_name><INSDQualifier_value>protein</INSDQualifier_value></IN
SDQualifier><INSDQualifier
id="q4"><INSDQualifier_name>organism</INSDQualifier_name><INSDQualifier_value>synth
etic
construct</INSDQualifier_value></INSDQualifier></INSDFeature_quals></INSDFeature><IN
SDFeature><INSDFeature_key>SITE</INSDFeature_key><INSDFeature_location>7..19</INS
DFeature_location><INSDFeature_quals><INSDQualifier
id="q56"><INSDQualifier_name>note</INSDQualifier_name><INSDQualifier_value>7</INSD
Qualifier_value><NonEnglishQualifier_value> The cysteine at position 7 is linked to the
cysteine at position 7 set forth in SEQ ID NO: 1 through an interchain disulfide bond
</NonEnglishQualifier_value></INSDQualifier><INSDQualifier
id="q57"><INSDQualifier_name>note</INSDQualifier_name><INSDQualifier_value>19</INS
DQualifier_value><NonEnglishQualifier_value> The cysteine at position 19 is linked to the
cysteine shown in SEQ ID NO: 1 through an interchain disulfide bond
</NonEnglishQualifier_value></INSDQualifier></INSDFeature_quals></INSDFeature><INSD

Feature><INSDFeature_key>MOD_RES</INSDFeature_key><INSDFeature_location>31</INS
DFeature_location><INSDFeature_quals><INSDQualifier
id="q17"><INSDQualifier_name>note</INSDQualifier_name><INSDQualifier_value>31</INS
DQualifier_value><NonEnglishQualifier_value> Lysine modified with PEG5CH2CO-gmma
Glu-eicosanedioic acid
</NonEnglishQualifier_value></INSDQualifier></INSDFeature_quals></INSDFeature></INSD
Seq_feature-table><INSDSeq_sequence>FVNQHLCGSHLVEALELVCGERGFHYTPKTX</I
NSDSeq_sequence></INSDSeq></SequenceData><SequenceData
sequenceIDNumber="4"><INSDSeq><INSDSeq_length>21</INSDSeq_length><INSDSeq_mol
type>AA</INSDSeq_moltype><INSDSeq_division>PAT</INSDSeq_division><INSDSeq_featu
re-table><INSDFeature><INSDFeature_key>source</INSDFeature_key><INSDFeature_locatio
n>1..21</INSDFeature_location><INSDFeature_quals><INSDQualifier><INSDQualifier_name
>mol_type</INSDQualifier_name><INSDQualifier_value>protein</INSDQualifier_value></IN
SDQualifier><INSDQualifier
id="q22"><INSDQualifier_name>organism</INSDQualifier_name><INSDQualifier_value>synt
hetic
construct</INSDQualifier_value></INSDQualifier></INSDFeature_quals></INSDFeature><IN
SDFeature><INSDFeature_key>DISULFID</INSDFeature_key><INSDFeature_location>6..11
</INSDFeature_location><INSDFeature_quals><INSDQualifier
id="q53"><INSDQualifier_name>note</INSDQualifier_name><INSDQualifier_value>6..11</I
NSDQualifier_value><NonEnglishQualifier_value> The cysteine at position 6 and the cysteine
at position 11 are linked by an intrachain disulfide bond
</NonEnglishQualifier_value></INSDQualifier></INSDFeature_quals></INSDFeature><INSD
Feature><INSDFeature_key>SITE</INSDFeature_key><INSDFeature_location>7..20</INSDFe
ature_location><INSDFeature_quals><INSDQualifier
id="q54"><INSDQualifier_name>note</INSDQualifier_name><INSDQualifier_value>7</INSD
Qualifier_value><NonEnglishQualifier_value> The cysteine at position 7 is linked to the
cysteine at position 7 shown in SEQ ID NO: 5 or SEQ ID NO: 6 through an interchain disulfide
bond </NonEnglishQualifier_value></INSDQualifier><INSDQualifier
id="q55"><INSDQualifier_name>note</INSDQualifier_name><INSDQualifier_value>20</INS

DQualifier_value><NonEnglishQualifier_value> The aspartic acid at position 20 is linked to the
X at position 19 shown in SEQ ID NO: 5 or SEQ ID NO: 6
</NonEnglishQualifier_value></INSDQualifier></INSDFeature_quals></INSDFeature></INSD
Seq_feature-table><INSDSeq_sequence>GIVEQCCTSICSLEQLENYDN</INSDSeq_sequence
></INSDSeq></SequenceData><SequenceData
sequenceIDNumber="5"><INSDSeq><INSDSeq_length>29</INSDSeq_length><INSDSeq_mol
type>AA</INSDSeq_moltype><INSDSeq_division>PAT</INSDSeq_division><INSDSeq_featu
re-table><INSDFeature><INSDFeature_key>source</INSDFeature_key><INSDFeature_locatio
n>1..29</INSDFeature_location><INSDFeature_quals><INSDQualifier><INSDQualifier_name
>mol_type</INSDQualifier_name><INSDQualifier_value>protein</INSDQualifier_value></IN
SDQualifier><INSDQualifier
id="q23"><INSDQualifier_name>organism</INSDQualifier_name><INSDQualifier_value>synt
hetic
construct</INSDQualifier_value></INSDQualifier></INSDFeature_quals></INSDFeature><IN
SDFeature><INSDFeature_key>MOD_RES</INSDFeature_key><INSDFeature_location>19..2
9</INSDFeature_location><INSDFeature_quals><INSDQualifier
id="q25"><INSDQualifier_name>note</INSDQualifier_name><INSDQualifier_value>29</INS
DQualifier_value><NonEnglishQualifier_value> X is lysine modified with
PEG5CH2CO-gamma Glu-eicosanedioic acid
</NonEnglishQualifier_value></INSDQualifier><INSDQualifier
id="q24"><INSDQualifier_name>note</INSDQualifier_name><INSDQualifier_value>19</INS
DQualifier_value><NonEnglishQualifier_value>X is
Dap</NonEnglishQualifier_value></INSDQualifier></INSDFeature_quals></INSDFeature><IN
SDFeature><INSDFeature_key>SITE</INSDFeature_key><INSDFeature_location>7..19</INS
DFeature_location><INSDFeature_quals><INSDQualifier
id="q51"><INSDQualifier_name>note</INSDQualifier_name><INSDQualifier_value>7</INSD
Qualifier_value><NonEnglishQualifier_value> The cysteine at position 7 is linked to the
cysteine at position 7 shown in SEQ ID NO: 4 through an interchain disulfide bond
</NonEnglishQualifier_value></INSDQualifier><INSDQualifier
id="q52"><INSDQualifier_name>note</INSDQualifier_name><INSDQualifier_value>19</INS

DQualifier_value><NonEnglishQualifier_value> X at position 19 is linked to the aspartic acid
shown in SEQ ID NO: 4
</NonEnglishQualifier_value></INSDQualifier></INSDFeature_quals></INSDFeature></INSD
Seq_feature-table><INSDSeq_sequence>FVNQHLCGSHLVEALELVXGERGFHYTPX</INS
DSeq_sequence></INSDSeq></SequenceData><SequenceData
sequenceIDNumber="6"><INSDSeq><INSDSeq_length>31</INSDSeq_length><INSDSeq_mol
type>AA</INSDSeq_moltype><INSDSeq_division>PAT</INSDSeq_division><INSDSeq_sequ
ence>FVNQHLCGSHLVEALELVXGERGFHYTPKTX</INSDSeq_sequence></INSDSeq></S
equenceData><SequenceData
sequenceIDNumber="7"><INSDSeq><INSDSeq_length>21</INSDSeq_length><INSDSeq_mol
type>AA</INSDSeq_moltype><INSDSeq_division>PAT</INSDSeq_division><INSDSeq_featu
re-table><INSDFeature><INSDFeature_key>source</INSDFeature_key><INSDFeature_locatio
n>1..21</INSDFeature_location><INSDFeature_quals><INSDQualifier><INSDQualifier_name
>mol_type</INSDQualifier_name><INSDQualifier_value>protein</INSDQualifier_value></IN
SDQualifier><INSDQualifier
id="q41"><INSDQualifier_name>organism</INSDQualifier_name><INSDQualifier_value>synt
hetic
construct</INSDQualifier_value></INSDQualifier></INSDFeature_quals></INSDFeature><IN
SDFeature><INSDFeature_key>MOD_RES</INSDFeature_key><INSDFeature_location>20</I
NSDFeature_location><INSDFeature_quals><INSDQualifier
id="q43"><INSDQualifier_name>note</INSDQualifier_name><INSDQualifier_value>20</INS
DQualifier_value><NonEnglishQualifier_value> X is L-4-bromo-2-aminobutyramide
</NonEnglishQualifier_value></INSDQualifier></INSDFeature_quals></INSDFeature><INSD
Feature><INSDFeature_key>DISULFID</INSDFeature_key><INSDFeature_location>6..11</I
NSDFeature_location><INSDFeature_quals><INSDQualifier
id="q45"><INSDQualifier_name>note</INSDQualifier_name><INSDQualifier_value>6..11</I
NSDQualifier_value><NonEnglishQualifier_value> The cysteine at position 6 and the cysteine
at position 11 are linked by an intrachain disulfide bond
</NonEnglishQualifier_value></INSDQualifier></INSDFeature_quals></INSDFeature><INSD
Feature><INSDFeature_key>SITE</INSDFeature_key><INSDFeature_location>7..20</INSDFe

ature_location><INSDFeature_quals><INSDQualifier
id="q48"><INSDQualifier_name>note</INSDQualifier_name><INSDQualifier_value>7</INSD
Qualifier_value><NonEnglishQualifier_value> The cysteine at position 7 is linked to the
cysteine at position 7 shown in SEQ ID NO: 8 or SEQ ID NO: 9 through an interchain disulfide
bond</NonEnglishQualifier_value></INSDQualifier><INSDQualifier
id="q47"><INSDQualifier_name>note</INSDQualifier_name><INSDQualifier_value>20</INS
DQualifier_value><NonEnglishQualifier_value> The X at position 20 is linked to the cysteine at
position 19 shown in SEQ ID NO: 8 or SEQ ID NO: 9
</NonEnglishQualifier_value></INSDQualifier></INSDFeature_quals></INSDFeature></INSD
Seq_feature-table><INSDSeq_sequence>GIVEQCCTSICSLEQLENYXN</INSDSeq_sequence
></INSDSeq></SequenceData><SequenceData
sequenceIDNumber="8"><INSDSeq><INSDSeq_length>29</INSDSeq_length><INSDSeq_mol
type>AA</INSDSeq_moltype><INSDSeq_division>PAT</INSDSeq_division><INSDSeq_featu
re-table><INSDFeature><INSDFeature_key>source</INSDFeature_key><INSDFeature_locatio
n>1..29</INSDFeature_location><INSDFeature_quals><INSDQualifier><INSDQualifier_name
>mol_type</INSDQualifier_name><INSDQualifier_value>protein</INSDQualifier_value></IN
SDQualifier><INSDQualifier
id="q42"><INSDQualifier_name>organism</INSDQualifier_name><INSDQualifier_value>synt
hetic
construct</INSDQualifier_value></INSDQualifier></INSDFeature_quals></INSDFeature><IN
SDFeature><INSDFeature_key>MOD_RES</INSDFeature_key><INSDFeature_location>29</I
NSDFeature_location><INSDFeature_quals><INSDQualifier
id="q62"><INSDQualifier_name>note</INSDQualifier_name><INSDQualifier_value>29</INS
DQualifier_value><NonEnglishQualifier_value> X is lysine modified with
PEG5CH2CO-gamma Glu-eicosanedioic acid
</NonEnglishQualifier_value></INSDQualifier></INSDFeature_quals></INSDFeature><INSD
Feature><INSDFeature_key>SITE</INSDFeature_key><INSDFeature_location>7..19</INSDFe
ature_location><INSDFeature_quals><INSDQualifier
id="q63"><INSDQualifier_name>note</INSDQualifier_name><INSDQualifier_value>7</INSD
Qualifier_value><NonEnglishQualifier_value>The cysteine at position 7 is linked to the cysteine

at position 7 of SEQ ID NO: 7 through an interchain disulfide bond
</NonEnglishQualifier_value></INSDQualifier><INSDQualifier
id="q64"><INSDQualifier_name>note</INSDQualifier_name><INSDQualifier_value>20</INS
DQualifier_value><NonEnglishQualifier_value> The cysteine at position 19 is linked to the X at
position 20 shown in SEQ ID NO: 7
</NonEnglishQualifier_value></INSDQualifier></INSDFeature_quals></INSDFeature></INSD
Seq_feature-table><INSDSeq_sequence>FVNQHLCGSHLVEALELVCGERGFHYTPX</INSD
Seq_sequence></INSDSeq></SequenceData><SequenceData
sequenceIDNumber="9"><INSDSeq><INSDSeq_length>31</INSDSeq_length><INSDSeq_mol
type>AA</INSDSeq_moltype><INSDSeq_division>PAT</INSDSeq_division><INSDSeq_featu
re-table><INSDFeature><INSDFeature_key>source</INSDFeature_key><INSDFeature_locatio
n>1..31</INSDFeature_location><INSDFeature_quals><INSDQualifier><INSDQualifier_name
>mol_type</INSDQualifier_name><INSDQualifier_value>protein</INSDQualifier_value></IN
SDQualifier><INSDQualifier
id="q66"><INSDQualifier_name>organism</INSDQualifier_name><INSDQualifier_value>synt
hetic
construct</INSDQualifier_value></INSDQualifier></INSDFeature_quals></INSDFeature><IN
SDFeature><INSDFeature_key>MOD_RES</INSDFeature_key><INSDFeature_location>31</I
NSDFeature_location><INSDFeature_quals><INSDQualifier
id="q69"><INSDQualifier_name>note</INSDQualifier_name><INSDQualifier_value>31</INS
DQualifier_value><NonEnglishQualifier_value> X is lysine modified with
PEG5CH2CO-gamma Glu-eicosanedioic acid
</NonEnglishQualifier_value></INSDQualifier></INSDFeature_quals></INSDFeature><INSD
Feature><INSDFeature_key>SITE</INSDFeature_key><INSDFeature_location>7..19</INSDFe
ature_location><INSDFeature_quals><INSDQualifier
id="q71"><INSDQualifier_name>note</INSDQualifier_name><INSDQualifier_value>19</INS
DQualifier_value><NonEnglishQualifier_value> The cysteine at position 19 is linked to the X at
position 20 shown in SEQ ID NO: 7
</NonEnglishQualifier_value></INSDQualifier><INSDQualifier
id="q70"><INSDQualifier_name>note</INSDQualifier_name><INSDQualifier_value>7</INSD

Qualifier_value><NonEnglishQualifier_value> The cysteine at position 7 is linked to the
cysteine at position 7 shown in SEQ ID NO: 7 through an interchain disulfide bond
</NonEnglishQualifier_value></INSDQualifier></INSDFeature_quals></INSDFeature></INSD
Seq_feature-table><INSDSeq_sequence>FVNQHLCGSHLVEALELVCGERGFHYTPKTX</I
NSDSeq_sequence></INSDSeq></SequenceData><SequenceData
sequenceIDNumber="10"><INSDSeq><INSDSeq_length>19</INSDSeq_length><INSDSeq_m
oltype>AA</INSDSeq_moltype><INSDSeq_division>PAT</INSDSeq_division><INSDSeq_se
quence>GIVEQCCTSICSLEQLENY</INSDSeq_sequence></INSDSeq></SequenceData><Seq
uenceData
sequenceIDNumber="11"><INSDSeq><INSDSeq_length>29</INSDSeq_length><INSDSeq_m
oltype>AA</INSDSeq_moltype><INSDSeq_division>PAT</INSDSeq_division><INSDSeq_fea
ture-table><INSDFeature><INSDFeature_key>source</INSDFeature_key><INSDFeature_locati
on>1..29</INSDFeature_location><INSDFeature_quals><INSDQualifier><INSDQualifier_nam
e>mol_type</INSDQualifier_name><INSDQualifier_value>protein</INSDQualifier_value></I
NSDQualifier><INSDQualifier
id="q74"><INSDQualifier_name>organism</INSDQualifier_name><INSDQualifier_value>synt
hetic
construct</INSDQualifier_value></INSDQualifier></INSDFeature_quals></INSDFeature><IN
SDFeature><INSDFeature_key>MOD_RES</INSDFeature_key><INSDFeature_location>19..2
9</INSDFeature_location><INSDFeature_quals><INSDQualifier
id="q79"><INSDQualifier_name>note</INSDQualifier_name><INSDQualifier_value>29</INS
DQualifier_value><NonEnglishQualifier_value> X is lysine modified with
PEG5CH2CO-gamma Glu-eicosanedioic acid
</NonEnglishQualifier_value></INSDQualifier><INSDQualifier
id="q86"><INSDQualifier_name>note</INSDQualifier_name><INSDQualifier_value>19</INS
DQualifier_value><NonEnglishQualifier_value>X is (S,S)
DAS[Dde-Asn(Trt)-OtBu]</NonEnglishQualifier_value></INSDQualifier></INSDFeature_qual
s></INSDFeature><INSDFeature><INSDFeature_key>SITE</INSDFeature_key><INSDFeatur
e_location>7..19</INSDFeature_location><INSDFeature_quals><INSDQualifier
id="q81"><INSDQualifier_name>note</INSDQualifier_name><INSDQualifier_value>19</INS

DQualifier_value><NonEnglishQualifier_value> The X at position 19 is linked to the tyrosine at
position 19 in SEQ ID NO: 10 </NonEnglishQualifier_value></INSDQualifier><INSDQualifier
id="q80"><INSDQualifier_name>note</INSDQualifier_name><INSDQualifier_value>7</INSD
Qualifier_value><NonEnglishQualifier_value> The cysteine at position 7 is linked to the
cysteine at position 7 shown in SEQ ID NO: 10 via an interchain disulfide bond
</NonEnglishQualifier_value></INSDQualifier></INSDFeature_quals></INSDFeature></INSD
Seq_feature-table><INSDSeq_sequence>FVNQHLCGSHLVEALELVXGERGFHYTPX</INS
DSeq_sequence></INSDSeq></SequenceData><SequenceData
sequenceIDNumber="12"><INSDSeq><INSDSeq_length>31</INSDSeq_length><INSDSeq_m
oltype>AA</INSDSeq_moltype><INSDSeq_division>PAT</INSDSeq_division><INSDSeq_fea
ture-table><INSDFeature><INSDFeature_key>source</INSDFeature_key><INSDFeature_locati
on>1..31</INSDFeature_location><INSDFeature_quals><INSDQualifier><INSDQualifier_nam
e>mol_type</INSDQualifier_name><INSDQualifier_value>protein</INSDQualifier_value></I
NSDQualifier><INSDQualifier
id="q83"><INSDQualifier_name>organism</INSDQualifier_name><INSDQualifier_value>synt
hetic
construct</INSDQualifier_value></INSDQualifier></INSDFeature_quals></INSDFeature><IN
SDFeature><INSDFeature_key>MOD_RES</INSDFeature_key><INSDFeature_location>19..3
1</INSDFeature_location><INSDFeature_quals><INSDQualifier
id="q89"><INSDQualifier_name>note</INSDQualifier_name><INSDQualifier_value>31</INS
DQualifier_value><NonEnglishQualifier_value> X is lysine modified with
PEG5CH2CO-gamma Glu-eicosanedioic acid
</NonEnglishQualifier_value></INSDQualifier><INSDQualifier
id="q88"><INSDQualifier_name>note</INSDQualifier_name><INSDQualifier_value>19</INS
DQualifier_value><NonEnglishQualifier_value>X is (S,S)
DAS[Dde-Asn(Trt)-OtBu]</NonEnglishQualifier_value></INSDQualifier></INSDFeature_qual
s></INSDFeature><INSDFeature><INSDFeature_key>SITE</INSDFeature_key><INSDFeatur
e_location>7..19</INSDFeature_location><INSDFeature_quals><INSDQualifier
id="q90"><INSDQualifier_name>note</INSDQualifier_name><INSDQualifier_value>7</INSD
Qualifier_value><NonEnglishQualifier_value> The cysteine at position 7 is linked to the

cysteine at position 7 shown in SEQ ID NO: 10 via an interchain disulfide bond
</NonEnglishQualifier_value></INSDQualifier><INSDQualifier
id="q91"><INSDQualifier_name>note</INSDQualifier_name><INSDQualifier_value>19</INS
DQualifier_value><NonEnglishQualifier_value> The X at position 19 is linked to the tyrosine at
position 19 shown in SEQ ID NO: 10
</NonEnglishQualifier_value></INSDQualifier></INSDFeature_quals></INSDFeature></INSD
Seq_feature-table><INSDSeq_sequence>FVNQHLCGSHLVEALELVXGERGFHYTPKTX</I
NSDSeq_sequence></INSDSeq></SequenceData></ST26SequenceListing>

## Claims

1. A compound, comprising:
(I) an amino acid sequence set forth in Formula I, 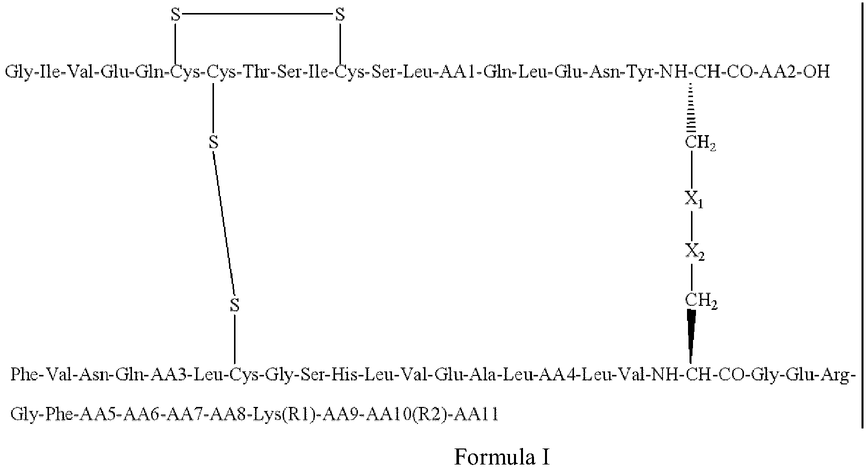 wherein,
X₁ in Formula I is selected from the group consisting of S, CH₃, NH and CO;
X₂ in Formula I is selected from the group consisting of S, CH₃, NH and CO;
AA1 in Formula I is selected from the group consisting of Asp, Glu and Ada;
AA2 in Formula I is selected from the group consisting of any amino acid for coding other than Cys, and any non-coded amino acid without SH group, or is absent;
AA3 in Formula I is selected from the group consisting of His, Tyr and Phe;
AA4 in Formula I is selected from the group consisting of Asp, Glu and Ada;
AA5 in Formula I is selected from the group consisting of His, Tyr and Phe;
AA6 in Formula I is selected from the group consisting of any amino acid for coding other than Cys, and any non-coded amino acid without SH group;
AA7 in Formula I is selected from the group consisting of any amino acid for coding other than Cys, and any non-coded amino acid without SH group;
AA8 in Formula I is selected from the group consisting of any amino acid for coding other than Cys, and any non-encodable amino acid without SH group;
AA9 in Formula I is Thr, or is absent;
AA10 in Formula I is selected from the group consisting of Lys, Dah, Orn, Dab and Dap, or is absent;
R1 and R2 in Formula I are HO₂C(CH₂)n1CO-(γGlu)n2-(PEGn3(CH₂)n4CO)n5-; and
R1 and R2 in Formula I are not present at the same time;
(II) an amino acid sequence obtained from substitution, deletion, addition and/or replacement of one or more amino acids of the amino acid sequence set forth in (I) ; or
(III) a sequence with more than 90% homology to the amino acid sequence set forth in (I) or (II); or
(IV) a pharmaceutically acceptable salt, solvate, chelate or non-covalent complex of the compound set forth in Formula I; and/or
(V) a drug precursor based on the compound shown in Formula I; and/or
(VI) a mixture comprising (I), (II), (III), (IV) and/or (V).

2. The compound according to claim 1, wherein,
when X₁ in Formula I is S, X₂ is S or CH₂;
when X₁ in Formula I is CH₂, X₂ is S or CH₂;
when X₁ in Formula I is NH, X₂ is CO;
when X₁ in Formula I is CO, X₂ is NH;
when X₁ and X₂ in Formula I are both S, AA11 is NH₂; and
when X₁ and X₂ in Formula I are not both S or both are not S, AA11 is NH₂ or OH.

3. The compound according to claim 1 or 2, wherein,
n1 is an integer selected from 10 to 25;
n2 is an integer selected from 1 to 5;
n3 is an integer selected from 1 to 30;
n4 is an integer selected from 1 to 5; and
n5 is an integer selected from 1 to 5.

4. The compound according to any one of claims 1 to 3, wherein the pharmaceutically acceptable salt thereof comprises a complex formed by the compound and Zn²⁺.

5. A method for producing the compound according to any one of claims 1 to 4, comprising:
Step 1: performing solid-phase polypeptide synthesis to obtain a peptide; and
Step 2: performing acid hydrolysis and purification to obtain the compound.

6. Use of any one of the following in the manufacture of a medicament or drug combination for preventing and/or treating a disease:
(I) the compound according to any one of claims 1 to 4; and/or
(II) the compound produced by the method according to claim 5.

7. Use of any one of the following in the manufacture of a medicament or drug combination for preventing and/or treating diabetes:
(I) the compound according to any one of claims 1 to 4; and/or
(II) the compound produced by the method according to claim 5;
wherein the diabetes includes type I diabetes, type II diabetes and/or gestational diabetes.

8. A drug, comprising the compound according to any one of claims 1 to 4 and/or the compound produced by the method according to claim 5, and an acceptable excipient and/or adjuvant.

9. A drug combination, comprising any one of the following and an additional active ingredient;
(I) the compound according to any one of claims 1 to 4; and/or
(II) the compound produced by the method according to claim 5; and/or
(III) the drug according to claim 8.

10. A method for preventing and/or treating diabetes, comprising administering any one of the following to a subject in need thereof:
(I) the compound according to any one of claims 1 to 4; and/or
(II) the compound produced by the method according to claim 5; and/or
(III) the drug according to claim 8; and/or
(IV) the drug combination according to claim 9;
wherein the diabetes includes type I diabetes, type II diabetes and/or gestational diabetes.
